(19) Europäisches Patentamt
European Patent Office
Office européen des brevets

(11) Publication number: **0 451 422 A1**

(12) **EUROPEAN PATENT APPLICATION**

(21) Application number: **90401022.0**

(51) Int. Cl.[5]: **C07C 229/36**, A61K 31/195

(22) Date of filing: **13.04.90**

(43) Date of publication of application: **16.10.91 Bulletin 91/42**

(84) Designated Contracting States: **FR**

(71) Applicant: **MERRELL DOW PHARMACEUTICALS INC.**
**2110 East Galbraith Road**
**Cincinnati Ohio 45215-6300(US)**

(72) Inventor: **McDonald, Ian A.**
**9382 Kentonsrun Court**
**Loveland, Ohio 45140(US)**
Inventor: **Jung, Michel J.**
**3 Place Echauffour, Engwiller**
**67350 Pfaffenhoffen(FR)**
Inventor: **Sabol, Jeffrey S.**
**1843 Poplar Drive**
**Loveland, Ohio 45140(US)**

(74) Representative: **Gillard, Marie-Louise et al**
**Cabinet Beau de Loménie 55, Rue d'Amsterdam**
**F-75008 Paris(FR)**

(54) **Pharmaceutically active fluoromethyltyrosine compounds.**

(57) This invention relates to novel monofluoromethyl- and difluoromethyltyrosine compounds which are active as tyrosine hydroxylase inhibitors and are therefore useful in the treatment of conditions caused by high levels of catecholamines such as hypertension, schizophrenia, and pheochromocytoma.



Rank Xerox (UK) Business Services

This invention relates to a compound of the formula

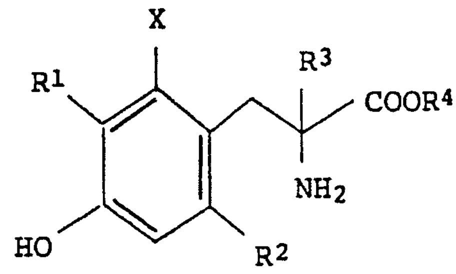

(I)

wherein
X is $CH_2F$ or $CHF_2$,
$R^1$ and $R^2$ are each independently hydrogen, fluorine, chlorine, bromine or iodine, and when one of either $R^1$ or $R^2$ is a halogen then the other must be hydrogen,
$R^3$ is hydrogen or methyl,
$R^4$ is hydrogen, $(C_1\text{-}C_4)$alkyl, phenyl or benzyl when X is $CHF_2$, and $R^4$ is hydrogen when X is $CH_2F$,
and to the salts thereof.

These compounds are useful as pharmaceutical agents for conditions which can be treated by the inhibition of tyrosine hydroxylase. Such conditions include hypertension, pheochromocytoma and schizophrenia.

In any compound within the scope of this invention, $R^1$ and $R^2$ can each be hydrogen, or either $R^1$ or $R^2$ can be any halogen when the other is hydrogen. The term "$(C_1\text{-}C_4)$alkyl" means saturated, straight or branched chain alkyl groups having from one to four carbon atoms, and includes such groups as methyl, ethyl, n-propyl, isopropyl, n-butyl, isobutyl, sec-butyl, and tert-butyl.

Since the compounds of Formula I possess an asymetric carbon atom, enantiomers are possible, and the compounds of the invention may be in the form of a biologically active individual enantiomer or mixtures of the enantiomers, such as the racemate. The compounds of Formula I may be obtained in the form of a pure enantiomer either by resolving a desired racemic product or by resolving a racemic starting material or intermediate at any convenient state of the synthesis. Methods of carrying out the resolution are well known in the art of chemistry. When dosage ranges are given herein, they are applicable to the racemate.

Illustrative examples of the compounds of this invention include:
2-fluoromethyl-p-tyrosine
2-difluoromethyl-p-tyrosine
α-methyl-2-fluoromethyl-p-tyrosine
α-methyl-2-difluoromethyl-p-tyrosine
2-fluoromethyl-3-fluoro-p-tyrosine
2-fluoromethyl-6-fluoro-p-tyrosine
2-difluoromethyl-3-fluoro-p-tyrosine
2-difluoromethyl-6-fluoro-p-tyrosine
α-methyl-2-fluoromethyl-3-fluoro-p-tyrosine
α-methyl-2-difluoromethyl-3-fluoro-p-tyrosine
2-fluoromethyl-3-chloro-p-tyrosine
2-fluoromethyl-6-chloro-p-tyrosine
2-difluoromethyl-3-chloro-p-tyrosine
2-difluoromethyl-6-chloro-p-tyrosine
α-methyl-2-difluoromethyl-3-chloro-p-tyrosine
α-methyl-2-difluoromethyl-6-chloro-p-tyrosine
α-methyl-2-difluoromethyl-3-bromo-p-tyrosine
α-methyl-2-difluoromethyl-6-bromo-p-tyrosine
α-methyl-2-difluoromethyl-3-iodo-p-tyrosine
α-methyl-2-difluoromethyl-6-iodo-p-tyrosine
α-methyl-2-fluoromethyl-3-chloro-p-tyrosine
α-methyl-2-fluoromethyl-6-chloro-p-tyrosine

α-methyl-2-fluoromethyl-3-bromo-p-tyrosine
α-methyl-2-fluoromethyl-6-bromo-p-tyrosine
α-methyl-2-fluoromethyl-3-iodo-p-tyrosine
α-methyl-2-fluoromethyl-6-iodo-p-tyrosine
2-difluoromethyl-p-tyrosine, ethyl ester

The compounds of this invention can be prepared using techniques well known in the art.

The monofluoromethyl compounds of formula I can be prepared by following the reactions depicted in Reaction Scheme I below:

REACTION SCHEME I

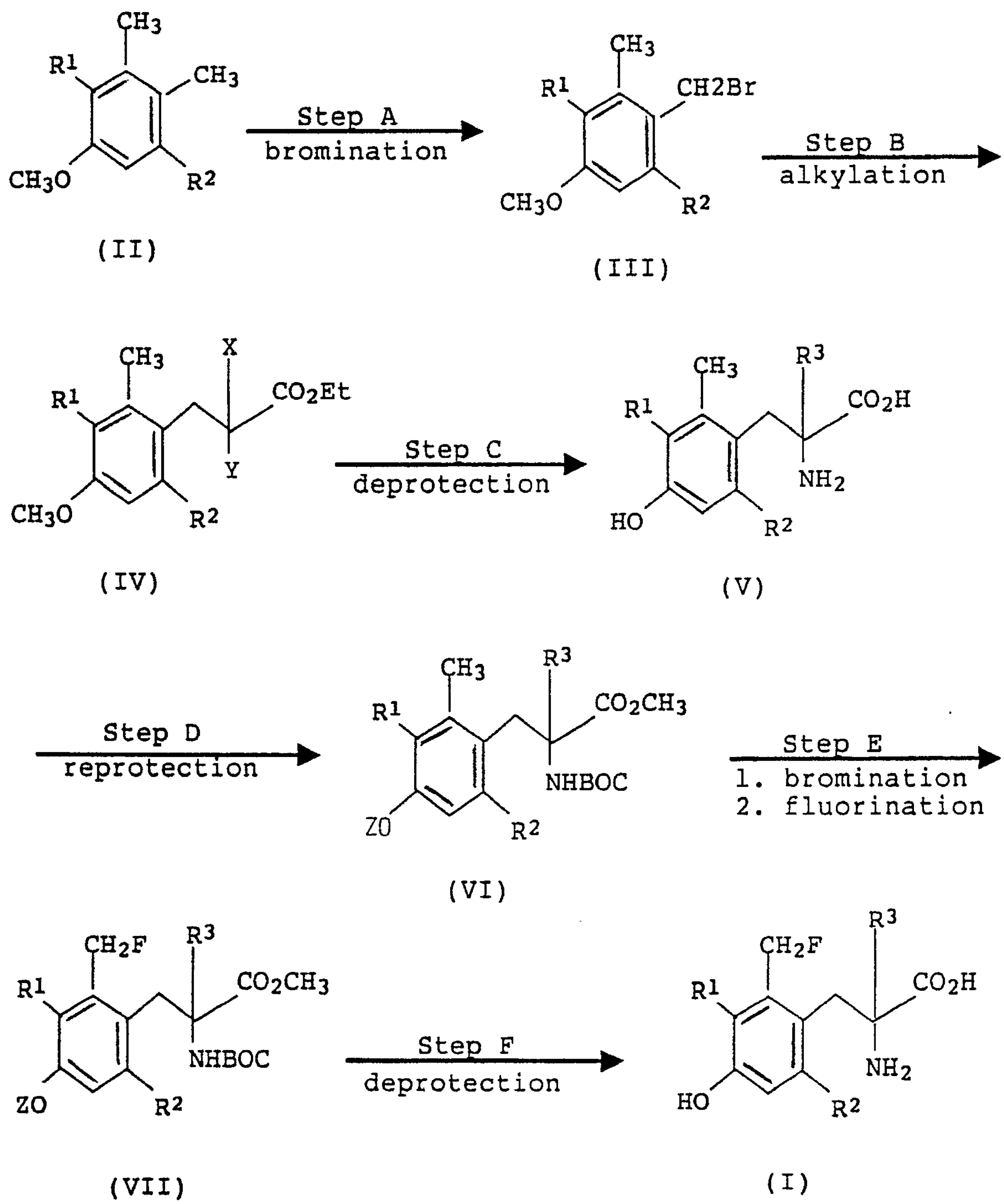


(II) (III) (IV) (V) (VI) (VII) (I)

wherein :

Z is butoxycarbonyl or acetyl, and wherein X is $CO_2Et$ or $R^3$ and Y is NHAc or $N=CHC_6H_5$ as long as when X is $CO_2Et$ then Y is NHAc and when X is $R^3$ then Y is $N=CHC_6H_5$, Ac being acetyl.

Each step of Reaction Scheme I is carried out using methods known in the art, unless otherwise specifically stated. Generally, Reaction Scheme I represents the following steps: Step A depicts bromination of the 1,2-dimethyl-4-methoxyphenylstarting material of Formula II to yield the brominated compound of Formula III in preparation for the alkylation reaction of Step B. In Step B, the brominated compound of Formula III is reacted with an alanine synthon to produce the protected amino acid compound of Formula IV which is deprotected in Step C by refluxing with hydrogen bromide and then neutralized with triethylamine to yield the 2-methyltyrosine compound of Formula V. In Step D, the carboxylic acid group of the Formula V compound is esterified by treatment with hydrogen chloride saturated methanol and the esterified compound is then treated with di-tert-butyl dicarbonate, triethylamine and dimethylaminopyridine to yield, after extraction and purification, the protected butoxycarbonyl methyltyrosine methyl ester of Formula VI. Alternatively, the protected acetoxy methyltyrosine methyl ester of Formula VI is prepared by treating the phenolic compound with an acetylating reagent, such as acetic anhydride, in the presence of dimethylaminopyridine.

Step E depicts the introduction of fluorine to the Formula VI compound which is accomplished by reaction of Formula VI first with N-bromosuccinimide (NBS) at reflux and then with a fluorination agent such as (AgF). Purification of the product yields the protected, 2-fluoromethyltyrosine compound VII, which is then deprotected, in Step F, by treatment with lithium hydroxide (LiOH), careful acidification, and extraction in ether. The acid is finally reacted with trifluoroacetic acid and treated with propylene oxide to yield the 2-fluoromethyltyrosine compounds of Formula I. Alternatively, the hydrochloride salt of the 2-fluoromethyltyrosine compounds described by Formula I can be prepared by treating the N-tert-butoxycarbonyl protected amino acid with a solution of hydrogen chloride in ether. For specific examples of this procedure, see Examples 1 through 7 below.

More specifically, Reaction Scheme I teaches how to prepare the monofluoromethyltyrosine compounds of Formula I by depicting the following synthetic steps:

In Step A, the dimethylanisole starting material described by Formula II is typically brominated by refluxing the Formula II compound with N-bromosuccinimide in a solvent such as carbon tetrachloride for about an hour or more, then recovering the brominated product (Formula III) by means of evaporation. The bromomethylanisole compound of Formula III is then converted, in Step B, to the amino acid protected ($X=CO_2Et$, $Y=NHAc$) compound described by Formula IV typically by means of a reaction run at room temperature for several hours, in a solvent such as dimethylformamide with either a preformed anion made from diethyl acetimidomalonate and sodium hydride or diethylacetimido malonate, and with subsequent organic solvent extraction and recrystallization. Alternatively, the anion derived from the Schiff base agent

$$EtO_2C\underset{\underset{R^3}{|}}{C}H-N=CHC_6H_5$$

can be similarly used as the alkylating agent to prepare the compound described by Formula IV ($X=R^3$ and $Y=N=CHC_6H_5$).

The compound described by Formula IV is then deprotected, as shown in Step C, by reaction with aqueous hydrogen bromide for several hours at reflux and subsequent neutralization with a base such as triethylamine or propylene oxide in a solvent such as ethanol to yield the 2-methyltyrosine compounds described by Formula V.

The esterification depicted as the first step in Step D is concerned with the esterification of the carboxylic acid group on the compound of Formula V, and is typically accomplished by treating the compound of Formula V with methanol in dry gaseous hydrochloric acid for about one or more hours at ambient temperature. The crude methyl ester is protected, as Step 2 of Step D, by treatment with at least two equivalents of di-tert-butyl dicarbonate and a base, such as triethylamine or 4-dimethylaminopyridine, in a solvent, such as THF or $CHCl_3$, at ambient temperature for three to twelve hours to yield the protected amino acid of Formula VI (Z=butoxycarbonyl). Alternatively, a two step procedure is used to prepare compounds of Formula VI in which Z=acetyl. Thus, treatment of the ester with one equivalent of di-tert-butyl dicarbonate in a two-phase $CHCl_3$/water reaction mixture with a suitable base, such as $NaHCO_3$, under reflux for one to six hours, followed by extraction and treatment of the crude phenol with an acetylating agent, such as acetic anhydride or acetyl chloride, in a solvent, such as $CH_2Cl_2$ or THF, in the presence of a base, such as 4-dimethylaminopyridine, afforded the differentially projected amino acid described by Formula VI.

The preparation of the fluorine-containing protected amino acid depicted by Formula VII is accomplished using a two step procedure. In the first step, the aromatic methyl group is brominated in the standard way using N-bromosuccinimide and benzoyl peroxide in a solvent, such as $CCl_4$ or $CH_2Cl_2$, under reflux for fifteen minutes to two hours. The crude bromomethyl compound is treated with a suitable fluorination reagent, such as AgF, in a solvent, such as $CHCl_3$ or $CH_3CN$, at ambient temperature for two to twelve hours. The pure protected fluoromethylamino acid described by Formula VII was obtained after silica chromatography.

The conditions for final Step F in this Scheme depends on the choice of protecting groups in compound VII. The protection amino acid described by Formula VII is de-esterified by the use of a base, such as LiOH, in a solvent, such as dimethoxyethane and water, at ambient temperature for one to six hours. When Z = butoxycarbonyl in Formula VII the two BOC groups are removed in a single step using trifluoroacetic acid at ambient temperature for fifteen to one-hundred twenty minutes. This procedure affords the trifluoroacetate salt of the compound described by Formula I.

When Z = acetyl in Formula VII, the acetyl group is removed in the basic hydrolysis step. The remaining BOC group is removed by treatment of the N-BOC protected amino acid with either trifluoroacetic acid or with anhydrous HCl in ether at ambient temperature for at least two hours. Either the trifluoroacetate salt or hydrochloride salt of the compound described by Formula I will be obtained depending on the conditions.

The compound described by Formula I can be purified as its hydrochloride salt or, preferably, the free amino acid is prepared by treatment of an alcoholic solution of either the hydrochloride or trifluoroacetate salt with a base, such as propylene oxide, at ambient temperature for one to twelve hours.

The starting materials for the synthetic route depicted in Reaction Scheme I, that is, the compounds of Formula II, are prepared by methods known in the art as depicted in Reaction Scheme II:

REACTION SCHEME II

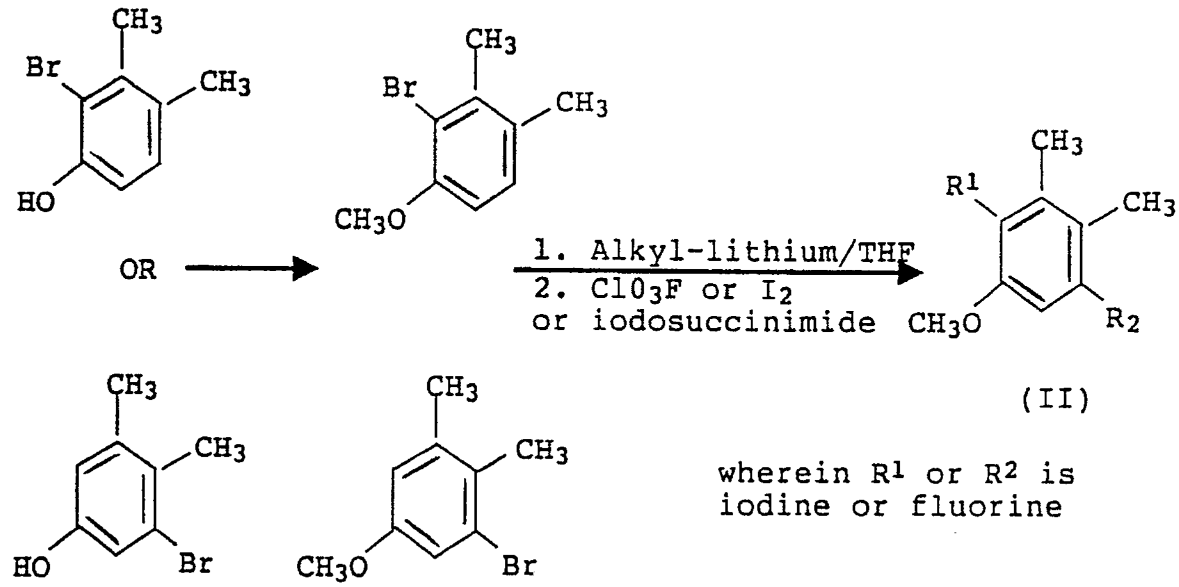


(II)

wherein $R^1$ or $R^2$ is iodine or fluorine

(II)

OR

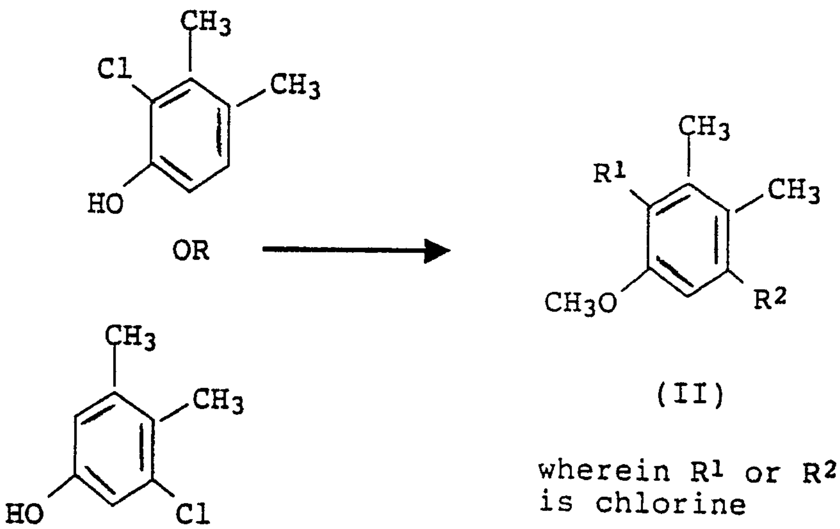


(II)

wherein $R^1$ or $R^2$ is chlorine

In essence Reaction Scheme II illustrates that the icdinated or fluorinated methoxy compounds of Formula II can be prepared from the corresponding brominated compounds in a two step procedure. Thus metal-halogen exchange is achieved using an alkylmetal reagent, such as n-butyllithium, sec-butyllithium or tert-butyllithium, in a solvent, such as ether or THF, cooled to -30° C to -78° C. The resulting aryl-lithium complex is quenched with a fluorinating reagent, such as $ClO_3F$, or an iodinating reagent, such as $I_2$ or N-iodosuccinimide, and the temperature is allowed to rise to ambient. The compounds of Formula II are extracted and purified by standard procedures. The chlorinated methoxy compounds of Formula II can be prepared by methylation of the known phenolic precursors by methods well known in the art.

The difluoromethyltyrosine compounds of Formula I can be prepared by following the reaction depicted in Reaction Scheme III below:

REACTION SCHEME III

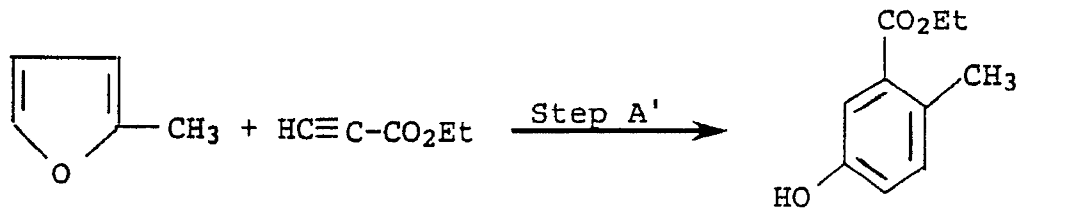


(VIII)

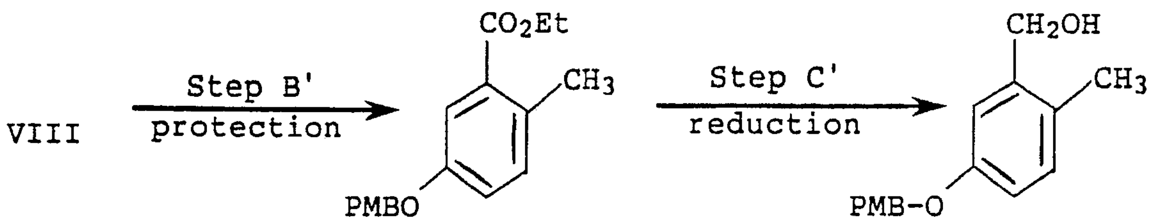


(IX)

(X)

REACTION SCHEME III - continued

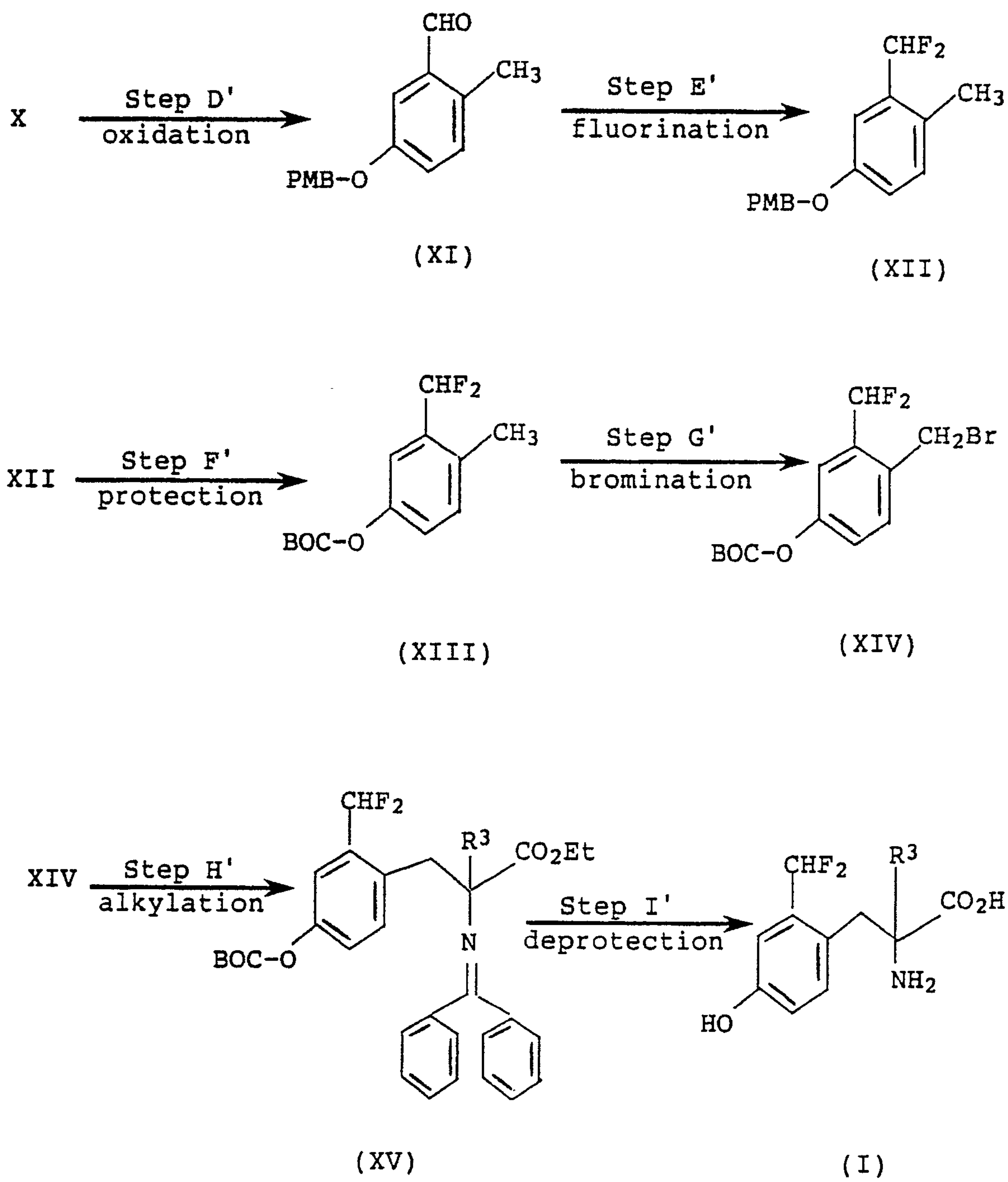


(XI) (XII) (XIII) (XIV) (XV) (I)

wherein PMB is para-methoxybenzyl and BOC is tert-butoxycarbonyl

Reaction Scheme III illustrates the general way in which difluoromethyl tyrosine compounds of Formula I in which $R_1$ and $R_2$ are hydrogen can be prepared. The preparation of compound VIII followed a published procedure by McCulloch and McInnes (Canad. J. Chem., 49, 3152 (1971). The phenol group in compound VIII is protected with a group, such as p-methoxybenzyl, by standard procedures. For example, a mixture of compound VIII in a solvent, such as acetone or THF, is treated with p-methoxybenzyl chloride in the presence of an activating agent, such as NaI, and a base, such as potassium carbonate, and refluxed for twelve to thirty-six hours. The resulting protected ester IX is reduced with a hydride reagent, such as lithium aluminum hydride or diisobutylaluminum hydride, in a non-polar solvent, such as hexane, toluene, THF, ether or $CH_2Cl_2$, at temperatures from ambient to reflux for one to twenty-four hours to yield the alcohol of Formula X. Oxidation to the aldehyde shown by Formula XI can be accomplished by standard selective

oxidation methods, such as the Swern oxidation in which the alcohol X is added to a reaction mixture cooled from -30 ° C to -78 ° C containing an acylating agent, such as oxalyl chloride, and dimethylsulfoxide in a solvent, such as $CH_2Cl_2$. The reaction proceeds for five to sixty minutes then is terminated by the addition of a base, such as triethylamine. Conversion of the aldehyde group in compound XI to the difluoromethyl group is achieved by reaction of compound XI with diethylaminosulfur trifluoride (DAST) in a solvent, such as $CH_2Cl_2$, for twelve to forty-eight hours to yield compound of Formula XII.

The protecting group in compound XII is exchanged for the tert-butoxycarbonyl group by a two step procedure. Removal of the p-methoxybenzyl group in compound XII is accomplished using a strong acid, such as trifluoroacetic acid, in a solvent, such as $CH_2Cl_2$, at ambient temperature for fifteen to one-hundred twenty minutes. The resulting phenol is reprotected with a suitable group, such as the tert-butoxycarbonyl group, by treatment with a suitable reagent, such as di-tert-butyl dicarbonate or 2-(tert-butoxycarbonyloxyimino)-2-phenylacetonitrile, in the presence of a base, such as 4-dimethylaminopyridine or triethylamine, at ambient temperature for one-half to six hours to afford compound described by Formula XIII. In step G' the aromatic methyl group in compound XIII is brominated using a brominating reagent, such as NBS, in a suitable solvent, such as $CCl_4$, $CHCl_3$, or $CH_2Cl_2$ under irradiation from a 300 W lamp for one-half to four hours to yield the bromomethyl compound XIV. Alkylation with a glycine or alanine synthon, such as the anion derived from N-(diphenylmethylene)glycine ethyl ester or N-(diphenymethylene)alanine ethyl ester, in a solvent, such as THF, ether or hexamethylphosphoramide, at -30 ° C to -70 ° C for one-half to six hours afforded the protected amino acid described by Formula XV. Hydrolysis of the acid and phenol protecting groups is achieved using an alkali metal base, such as lithium hydroxide, in a solvent, such as dimethoxyethane or THF mixed with water, at ambient temperature for four to twenty-four hours. Removal of the N-protecting group is achieved under acidic conditions, such as treatment with aqueous HCl and ether in a two phase system. The final part of Step I' is to treat the resulting hydrochloride salt of I with a suitable base, such as triethylamine or propylene oxide, to yield the free difluoromethyl amino acids described by Formula I in which $R^1$ and $R^2$ are both hydrogen.

The esters of the difluoromethyltyrosine structures of Formula I that is, the compounds wherein $R^4$ is other than H, and X = difluoromethyl, can be prepared by procedures well known in the art. In general, a mixture of the free amino acid in an alcohol $R^4OH$, such as methanol, ethanol, or benzyl alcohol, is treated with an acid, such as sulfuric acid or hydrogen chloride, at ambient temperatures for one-half to twelve hours. A co-solvent, such as toluene or $CH_2Cl_2$, may also be employed. Extraction and purification yields the esters described by Formula I as their salts, preferably as hydrochloride salts.

REACTION SCHEME IV

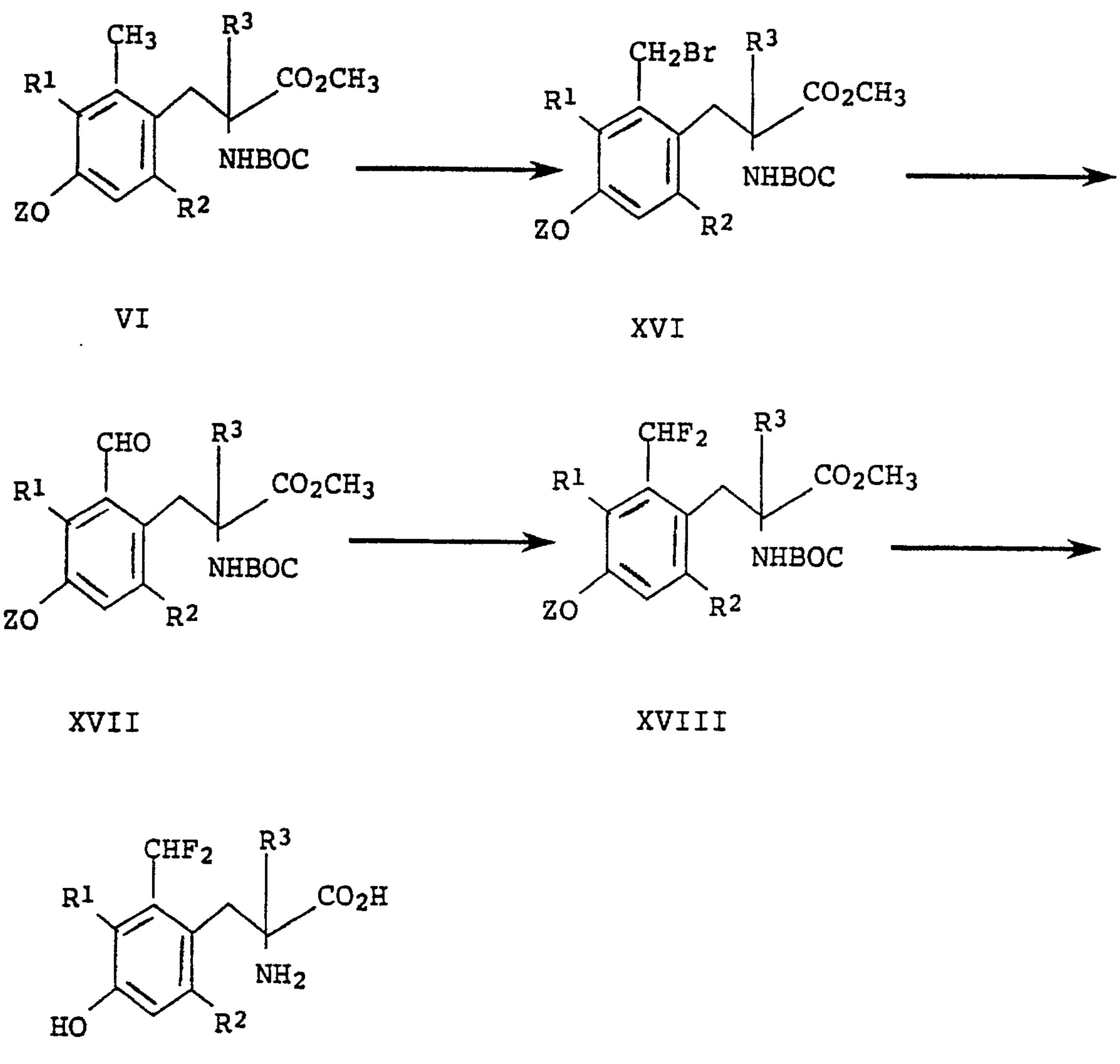

VI

XVI

XVII

XVIII

I

Reaction Scheme IV illustrates a general preparation of difluoromethyltyrosine derivatives. The protected amino acid VI is described in Scheme I. Bromination as described above affords the bromomethyl compound shown in Formula XVI. Oxidation of the bromomethyl group can be achieved by a number of standard procedures, such as the Sommelet reaction or treatment with $AgF_4$ and DmSO, to yield the aldehyde XVII.

Conversion of compound XVII to I comprises the conversion of compound XVII to XVIII according to step E' (fluorination) of reaction Scheme III and the conversion of compound XVIII to I according to step F (deprotection) of reaction Scheme I.

The following specific examples are presented to illustrate the synthesis of the compounds of this invention, but they should not be construed as limiting the scope of this invention in any way.

EXAMPLE 1

2-Methyltyrosine (V)

A mixture of 3,4-dimethylanisole (II) (16.80g) and N-bromosuccinimide (22.00g) in $CCl_4$ (740 ml) was refluxed for one hour, then cooled, washed with water, dried and evaporated. A solution of this product in dimethylformanide (DMF, 70 ml) was added to a preformed anion made from diethyl acetimidomalonate

(26.80g) and NaH (5.93g) in DMF (300 ml). After the addition, the reaction mixture was stirred for sixteen hours, then diluted with water and extracted with ether. Recrystallization of the resulting product from $CH_2Cl_2$/hexane afforded 23.19g of compound IV as colorless crystals; mp 102-103 °C.

A mixture of IV (10.00g) in aqueous HBr(40%, 300 ml) was refluxed for five hours then evaporated to dryness. Ethanol (100 ml) was added, then the solution was neutralized with triethylamine (4.0 ml). Evaporation followed by recrystallization from aqueous ethanol yielded 2.27g of title compound as colorless crystals; mp 245-249 °C (dec). 2-Methyltyrosine has been previously reported by H. Schmalfuss and W. Resche in Ber, 62B, 2591, (1929).

EXAMPLE 2

N,O-tert-Butoxycarbonyl 2-Methyltyrosine, Methyl Ester (VI)

A solution of 2-methyltyrosine (V) from Example I (27.8g) in $CH_3OH$/dry gaseous HCl (600 ml) was treated with 2,2-dimethoxypropane then stirred at ambient temperature overnight. The solvents were evaporated and the residue was dissolved in water to which solid $NaHCO_3$ was added to adjust the pH to about eight. Ethyl acetate extraction followed by filtration through silica afforded 14.64g of a yellowish solid.

A slurry of this product in tetrahydrofuran (THF, 600 ml) was treated with di-tert-butyl dicarbonate (30.54g), triethylamine (9.8 ml) and a few crystals of 4-dimethylaminopyridine then stirred at ambient temperature for three hours. The solvent was evaporated and the residue was taken up in $CH_2Cl_2$, then washed with water, dried and evaporated. Flash chromatography on silica, using an eluant of 60% hexane/40% ethyl acetate afforded pure title compound (24.66g) as a colorless oil.

EXAMPLE 3

N,O-tert-Butoxycarbonyl 2-Fluoromethyltyrosine, methyl ester (VII)

A solution of the compound (VI) of Example 2 (16.0g) and N-bromosuccinimide (7.66g) in $CH_2Cl_2$ (150 ml) and $CCl_4$ (450 ml) was refluxed (300 W lamp) for 1.25 hours. After cooling, the solution was washed with water, 4% aq. $NaHCO_3$ and water, dried and evaporated to leave a yellow foam. This foam was dissolved in $CH_3CN$ (280 ml), AgF (5.45g) was added and the mixture was stirred in the absence of light at 55 °C for two hours. EtOAc (600 ml) was added and the mixture was passed through a short column of celite. Evaporation of the solvents left a foam (14.5g) from which pure title compound (2.49g) was recovered by preparative high pressure liquid chromatography (hexane/acetone mixtures) followed by recrystallization. The analytically pure material crystallized as colorless needles; mp 73-75 °C.

EXAMPLE 4

2-Fluoromethyltyrosine (I)

A solution of the compound (VII) of Example 3 (1.89g) and lithium hydroxide monohydrate (1.40g) in dimethoxyethane (200 ml) and water (40 ml) was stirred at ambient temperature for 1.75 hours. The solution was diluted with water (400 ml), washed twice with ether, then acidified to about pH 3 with 10% aq. HCl. Ether extraction in the usual way afforded the crude acid as a colorless viscous oil (1.80g). This product was stirred for thirty minutes with trifluoroacetic acid (50 ml) then evaporated to leave a viscous oil (2.03g). This substance was treated overnight at ambient temperature with propylene oxide (25 ml) and isopropanol (25 ml) whereupon the title compound (0.58g) formed as a colorless powder, containing a small amount of isoproperol; mp, decomposes above 205 °C; CHN elemental analysis, NMR ($D_2O$) $\delta$5.45, d(J = 47 Hz).

A synthetic route for producing 2-fluoromethyltyrosine as its hydrochloride salt is illustrated by specific Examples 5 through 7.

EXAMPLE 5

N-tert-Butoxycarbonyl, O-acetyl 2-Methyltyrosine, methyl ester

A solution of 2-methyltyrosine (V) of Example 1 (10g) in $CH_3OH$/dry HCl (250 ml) was left for three hours at room temperature, then evaporated to dryness. The crude methyl ester was dissolved in water (150 ml), treated with solid $NaHCO_3$ (to give pH 8) and solid NaCl (40g), then a $CHCl_3$ (200 ml) solution of

di-tert-butyl dicarbonate (11.18g) was added and the mixture was refluxed for three hours. The organic layer was separated, the aqueous layer extracted with $CHCl_3$, then the combined $CHCl_3$ extracts were washed with water, dried and evaporated to leave a light brown solid (13.2g). A solution of this substance in $CH_2Cl_2$ (200 ml) was treated with acetic anhydride (4.71 ml) and 4-dimethylaminopyridine (6.1g). After stirring overnight at room temperature the solution was washed with dilute aqueous HCl, then with water, then dried and evaporated to leave a semi-solid residue. Recrystallization afforded pure title compound (5.74g).

EXAMPLE 6

N-tert-Butoxycarbonyl,O-Acetyl 2-Fluoromethyltyrosine, Methyl Ester (VII)

A mixture of the compound (VI) of Example 5 (1.00g), benzoyl peroxide (25.0 mg) and N-bromosuccinimide (0.60g) in $CH_2Cl_2$ (40 ml) was refluxed for one hour, cooled, washed with water, dried and evaporated. The residue was dissolved in $CH_3CN$ (40 ml), AgF (0.38g) was added and the mixture was stirred out ambient temperature for three hours. Ethylacetate (10 ml) was added and the mixture was passed through a short column of neutral alumina using ethyl acetate as eluant. Evaporation of the solvents left a viscous oil (1.10g) from which essentially pure title compound (0.17g) was obtained after silica chromatography. Recrystallization from $CH_2Cl_2$/petroleum ether afforded the title compound as colorless needles; mp 111-112° C.

EXAMPLE 7

2-Fluoromethyltyrosine hydrochloride (I)

A solution of the compound (VII) of Example 6 (115 mg) and lithium hydroxide monohydrate (39.0 mg) in dimethoxyethane (5 ml) and water (1 ml) was stirred for two hours at room temperature. The solution was diluted with water then washed with ether. Careful acidification with dilute aq. HCl to pH 3 followed by ether extraction in the usual way yielded a waxy solid (106.0 mg). This material was taken up into HCl saturated ether (20 ml) and stirred for two days. A colorless precipitate slowly formed; filtration afforded the title compound (20.0 mg). MS (of the methyl ester, methyl ether, N-trifluoroacetyl) : $M+$ = 337 (Calc. 337). A second sample (199.0 mg) prepared in the same manner, had mp 130° C (dec) and NMR ($CDCl_3$) : $\delta$5.33, d (J = 48 HZ), $CH_2F$.

In like manner, by substituting the following compounds for 3,4-dimethylanisole of Example 1 (i.e., a compound of Formula II), and following the procedure set forth in Examples 1 through 7, the following compounds can be made:

Start with 2-chloro-3,4-dimethylanisole to yield 2-fluoromethyl-3-chloro-p-tyrosine; start with 2-fluoro-3,4-dimethylanisole to yield 2-fluoromethyl-3-fluoro-p-tyrosine; start with 3,4-dimethyl-5-chloroanisole to yield 2-fluoromethyl-6-chloro-p-tyrosine; start with 3,4-dimethyl-5-fluoroanisole to yield 2-fluoromethyl-6-fluoro-p-tyrosine.

EXAMPLE 8

3-Carboethoxy-4-methylphenol (VIII)

The preparation according to the process set forth in McCulloch, A.W.; McInnes, A.G. Can. J. Chem., 49, 3152-7, 1971 was used. A solution of 2-methylfuran (41.82g, 0.51 mol,46 mL) in $CH_2Cl_2$ (500 mL) was added at about 20° C to a mixture of ethyl propiolate (50g, 0.51 mol.) and anhydrous $AlCl_3$ (67.84, 0.51 mol.) in $CH_2Cl_2$ (2500 mL). After stirring for thirty minutes at room temperature, the mixture was then shaken vigorously with cold water. The organic layer was separated, dried over $MgSO_4$, and evaporated to a dark oil. The oil was poured onto silica gel (600 mL) and eluted with 5% ethylacetate-hexane and then 20% ethyl acetate-hexane. The 20% ethylacetate-hexane fractions were evaporated to dryness. The crude solid was recrystallized from chloroform/hexane to afford the title compound (19.1g, 0.106 mol., 21%), m.p. 57-59°.

EXAMPLE 9

1-(4-Methoxybenzyloxy)-3-carboethoxy-4-methylbenzene (IX)

A mixture of the compound (VIII) of Example 8 (17.1g, 0.095 mol.) anhydrous potassium carbonate (13.13g, 0.095 mol.), sodium iodide (13.68g, 0.091 mol.) and p-methoxybenzyl chloride (14.72g, 0.094 mol.) in acetone (600 mL) was heated at reflux for twenty-four hours. The mixture was cooled and the solids filtered off. The solvent was evaporated and the residue slurried with 2% ethyl acetate-hexane and poured onto silica gel (600 mL) and eluted first with 2% ethyl acetate-hexane (1 L) then 7% ethyl acetate-hexane (2 X 500 mL) and 25% ethyl acetate-hexane (2 X 500 mL). The second 7% ethyl acetate-hexane fraction was evaporated to a solid which was recrystallized from hexane-chloroform to afford of title compound (IX) (10.76g, 0.036 mol., 38%), m.p. 53-55° C.

EXAMPLE 10

1-(4-Methoxybenzyloxy)-3-hydroxymethyl-4-methylbenzene (X)

To a solution of 1.5M diisobutylaluminum hydride (DIBAL) in toluene (50 mL, 0.075 mol.) in toluene (200 mL) under argon and cooled in an ice-water bath, was added compound IX of Example 9 (10.69g, 0.0356 mol.) in toluene (25 mL) dropwise via syringe. The ice-water bath was removed and stirring was continued for one hour at room temperature. The mixture was again cooled in an ice-water bath and water (40 mL) was carefully added dropwise with stirring. The bath was removed and stirring was continued for thirty minutes at room temperature. The solid was filtered off through Celite and washed with ether. The filtrate was evaporated to a solid which was filtered on to a Buchner funnel with hexane and air dried to afford the title compound X (8.72g, 0.0338 mol., 95%); m.p. 73-75°.

EXAMPLE 11

1-(4-Methoxybenzyloxy)-3-formyl-4-methylbenzene (XI)

A solution of 2.0M methylene chloride solution of oxalyl chloride (23.4 mL, 0.047 mol.) in methylene chloride (125 mL) was cooled to -70° C in a dry ice-acetone bath. Dimethyl sulfoxide (0.094 mol., 6.64 mL) was added dropwise via syringe keeping the temperature below -55° C and the mixture was then stirred for ten minutes at -70° C under Argon. Compound X of Example 10 (8.64g, 0.033 mol.) in methylene chloride (25 mL) was added dropwise over a five minute period and the mixture was then stirred for twenty minutes at -70° C. Triethylamine (0.165 mol., 22.9 mL) was added dropwise via syringe, the bath was removed and the mixture was allowed to warm to room temperature over a forty-five minute period. The solid was then filtered off and the filtrate evaporated to dryness. The crude product was filtered through silica gel with ether, and the solvent removed to afford a solid which was washed onto a Buchner funnel with hexane and air dried to yield the title compound (7.04g, 0.0275 mol., 82%), m.p. 74-76° C.

EXAMPLE 12

1-( 4-Methoxybenzyloxy)-3-difluoromethyl-4-methylbenzene (XII)

A solution of compound XI of Example 11 (7.04g, 0.0275 mol.) in methylene chloride (50 mL) was added to diethylaminosulfur trifluoride (DAST, 0.04 mol., 5.28 mL) in methylene chloride (100 mL), and stirring was continued under argon at room temperature for twenty-four hours. The solution was then partitioned between ice water-ether and the organic layer was separated, dried over $MgSO_4$, filtered and evaporated to dryness. Chromatography, 5-7% ethyl acetate-hexane, afforded the title compound XII (6.68g, 0.024 mol., 90%), m.p. 87-89° C.

EXAMPLE 13

1-tert-Butoxycarbonyloxy-3-difluoromethyl-4-methylbenzene (XIII)

Trifluoracetic acid (10 mL) was added to a solution of compound XII of example 12 (0.024 mol.) in methylene chloride (50 mL) at room temperature under argon, and the reaction mixture was stirred for one-half hour. The methylene chloride and trifluoracetic acid were removed and the reside purified by preparative liquid chromatography, 15% ethyl acetate-hexane, to afford an intermediate phenol compound (3.2g, 0.02 mol., 83%) as an oil.

To this phenol compound (0.02 mol.) in THF (80 mL) was added di-tert-butyl dicarbonate (4.37g, 0.02

mol.) in THF (20 mL), followed by 4-dimethylaminopyridine (0.15g) and triethylamine (0.02 mol., 2.79 mL). Stirring was continued for one hour at room temperature and the solvent was thenremoved. The residue was filtered through silica gel with 5% ethyl acetate-hexane and the solvent was removed to afford the title compound (4.06g, 0.0157 mol., 78.5%) as an oil.

EXAMPLE 14

1-tert-Butoxycarbonyloxy-3-difluoromethyl-4-bromomethyl Benzene (XIV)

A solution of compound XIII of example 13 (3.95g, 0.0153 mol.) and N-bromosuccinimide (3.03g, 0.017 mol.) in $CCl_4$ (125 mL) was maintained at reflux under irradiation with a lamp for one and one-half hours. The mixture was cooled, the solid filtered off and the filtrate poured into saturated aqueous bicarbonate. The organic layer was separated, dried over $MgSO_4$, filtered and evaporated to dryness. Preparative liquid chromatography, 2.5% ethyl acetate-hexane, afforded recovered compound XIII of Example 13 (0.8g, 0.003 mol., 20.2%) followed by the title compound XIV (3.6g, 0.0107 mol., 70%) as an oil.

EXAMPLE 15

O-Butoxycarbonyl, N-Diphenylmethylene 2-Difluoromethyltyrosine, Ethyl Ester (XV)

To 1M lithium bis(trimethylsilyl)amide in THF (9.1 mL) in THF (80 mL) under argon at -70° C was added hexamethylphosphoramide (0.012 mol., 2 mL) followed by a solution of N-(diphenylmethylene)glycine ethyl ester (2.41 g, 0.009 mol.) in THF (10 mL) and the brown-yellow solution stirred at -70° C for one hour. A solution of compound XIV of example 14 (3.4g, 0.01 mol.) in THF (10 mL) was added via syringe and the solution was stirred for one hour at -70° C. The mixture was poured in to ice-water and then diluted with ether. The organic layer was separated, washed with saturated aqueous brine and then dried over $MgSO_4$, filtered and evaporated. The residue was purified by preparative liquid chromatography, 10% ethyl acetate-hexane to afford the title compound XV (3.437g, 0.0066 mol., 73%) as an oil.

EXAMPLE 16

2-Difluoromethyltyrosine (I)

A solution of lithium hydroxide monohydrate (0.96g, 22.9 mmol.) in water (25 ml) was added to a solution of compound XV of Example 15 (1.67g, 3.18 mmol.) in 1,2-dimethoxyethane (25 ml) and stirring was continued for 16 hours at room temperature. The mixture was then partitioned between ether (100 ml) and water (25 ml) and the aqueous layer was separated, cooled in an ice-water bath and then acidified with 3 N HCl (8 ml). The product was extracted into ethyl acetate, dried over $MgSO_4$, filtered and evaporated to an oil which solidified when dried on the vacuum pump. The solid was slurried with $CHCl_3$, filtered and dried.

This material (0.59g, 1.49 mmol.) was stirred in a two-phase mixture of IN HCl (7 mL) and ether (10 mL) at room temperature for sixteen hours. The phases were then separated and the aqueous layer was washed twice with ether. The aqueous layer was evaporated to dryness and the resulting solid was slurried with $CHCl_3$ and filtered whereupon the hydrochloride salt of 2-difluoromethylenetyrosine was obtained (0.34g).

The hydrochloride salt was dissolved in isopropanol (15 mls), propylene oxide (15 mls) was added, and the solution was kept at room temperature for two hours during which time precipitation occurred. The solid was collected washed with $CHCl_3$, and air dried to afford the title compound as a white powder (0.20g) mp 235-238° C. $^1$HNMR (300 MHz, $D_2O$) $\delta$3.1(1H, dd), 3.35 (1H, dd), 3.9(1H,m), 6.9 (t,$J_{H\text{-}F}$ = 55Hz) $^{19}$FNMR (282 MHz, $D_2O$) $\delta$-109.528, -109.723

In like manner, by substituting N-(diphenylmethylene)alanine ethyl ester for N-(diphenylmethylene) glycine ethyl ester of Example 15, and following the procedure set forth in Example 15 through 16, $\alpha$-methyl-2-difluoromethyltyrosine can be made.

The compounds of this invention are useful as tyrosine hydroxylase inhibitors for the treatment of, for instance, hypertension, pheochromocytoma, or schizophrenia. Tyrosine hydroxylase plays a major role in the catecholamine pathway by facilitating the conversion of tyrosine to dopa. This conversion is generally regarded as the rate limiting step in the syntheses of the catecholamine neurotransmitters. Inhibition of tyrosine hydroxylase causes a decrease in the amount of norepinephrine produced, which, in turn, effectuates a lowering of blood pressure. Additionally, inhibition of tyrosine hydroxylase causes a decrease

in the amount of dopamine produced which, in turn, effectuates a desired antischizophrenic effect. Similarly, inhibition of tyrosine hydroxylase causes a decrease in the availability of the catecholamine hormones or precursors which is effective in the treatment of hypertension associated with pheochromocytoma.

The tyrosine hydroxylase inhibitory properties of the compounds of this invention can readily be determined by standard and well known procedures. For example, determination of tyrosine hydroxylase inhibition is exemplified by a procedure wherein mice are injected intraperitoneally with a test compound, and six hours later the mice are sacrificed, the cerebral cortices removed and frozen on dry ice. The levels of norepinephrine in the tissue are measured by HPLC with electrochemical detection, according to the process of Dudley, et al, J. Pharmacol. Exp. Ther., 217, 834-840 (1981). Reduction in cortical norepinephrine levels indicates effective, in vivo inhibition of tyrosine hydroxylase. The following Table I illustrates the decreased levels of norepinephrine after treatment with a compound of this invention (2-fluoromethyl tyrosine) in relation to levels of norepinephrine present after treatment with a known inhibitor (alpha-methyl-p-tyrosine), and as compared to the amount of norepinephrine produced without treatment with a test compound (control).

TABLE I

IN VIVO INHIBITION OF TYROSINE HYDROXYLASE

| TEST COMPOUND | NOREPINEPHRINE | PERCENT OF CONTROL |
|---|---|---|
| Control | 198.2 +/- 18.4 ng/g | 100% |
| α-methyl-p-tyrosine | 115.7 +/- 18.6 ng/g | 58 +/- 9% |
| 2-fluoromethyl-tyrosine | 124.9 +/- 11.4 ng/g | 63 +/- 6% |

A patient for the purpose of this invention is a mammal, including a human, in need of treatment for a particular condition, injury or disease such as hypertension, schizophrenia or pheochromocytoma. The effective dosage amount of the compounds of this invention to be administered to patients can readily be determined by comparison of the results from standard pharmacological assays and standard toxicity tests, with the results of known pharmacological agents.

Additionally, the amount of active ingredient (i.e., a compound of Formula I) to be administered to a patient for the treatment of either hypertension, schizophrenia or pheochromocytoma can vary widely according to such considerations as the particular compound and dosage unit employed, the period of treatment, the age and sex of the patient treated, and the type and extent of the hypertension or schizophrenia treated.

The total amount of active ingredient to be administered parenterally for treatment of hypertension, schizophrenia or pheochromocytoma will generally range from about 0.1 mg/kg to 30 mg/kg and preferably from 1.0 mg/kg to 10.0 mg/kg. A unit dosage may contain from 5 mg to 525 mg of active ingredient, and can be taken one or more times per day. For example, a 50 kg patient may be administered 50 mg - 700 mg active ingredient four times a day for a total dose of 200 mg - 2800 mg per day.

The total amount of active ingredient to be administered orally for the treatment of hypertension, schizophrenia or pheochromocytoma will generally range from 0.1 mg/kg to 100 mg/kg, and preferably from 1.0 mg/kg to 50 mg/kg. A unit dosage may contain from 5 mg to 1000 mg of active ingredient, and can be taken one or more times per day. For example, a 50 kg patient may be administered 50 mg - 2500 mg of active ingredient four times a day for a total of 200 mg - 10,000 mg per day.

The compounds of this invention can be utilized to achieve the desired pharmacological effect by administration to a patient in need thereof in an appropriately formulated pharmaceutical composition. Therefore, the present invention includes pharmaceutical compositions which are comprised of a pharmaceutically-acceptable carrier and a pharmaceutically-effective amount of a compound of Formula I. A pharmaceutically-acceptable carrier is any carrier which is relatively non-toxic and innocuous to a patient at concentrations consistent with effective activity of the active ingredient so that any side effects ascribable to the carrier do not vitiate the beneficial effects of the active ingredient. A pharmaceutically-effective amount of compound is that amount which produces a result or exerts an influence on the particular condition being treated. The compounds of Formula I can be administered with a pharmaceutically-acceptable carrier using conventional dosage unit forms orally, parenternally, topically, as an aerosol, or the like.

For oral administration, the compounds can be formulated into solid or liquid preparations such as capsules, pills, tablets, troches, lozenges, melts, powders, solutions, suspensions, or emulsions and may be prepared according to methods known to the art for the manufacture of pharmaceutical compositions. The solid unit dosage forms can be a capsule which can be of the ordinary hard- or soft-shelled gelatin type containing, for example, surfactants, lubricants, and inert fillers such as lactose, sucrose, calcium phosphate, and cornstarch.

In another embodiment, the compounds of this invention may be tableted with conventional tablet bases such as lactose, sucrose, and cornstarch in combination with binders such as acacia, cornstarch, or gelatin, disintegrating agents intended to assist the break-up and dissolution of the tablet following administration such as potato starch, alginic acid, corn starch, and guar gum, lubricants intended to improve the flow of tablet granulations and to prevent the adhesion of tablet material to the surfaces of the tablet dies and punches, for example, talc, stearic acid, or magnesium, calcium, or zinc stearate, dyes, coloring agents, and flavoring agents intended to enhance the aesthetic qualities of the tablets and make them more acceptable to the patient. Suitable excipients for use in oral liquid dosage forms include diluents such as water and alcohols, for example, ethanol, benzyl alcohol, and the polyethylene alcohols, either with or without the addition of a pharmaceutically acceptably surfactant, suspending agent, or emulsifying agent.

The compounds of this invention may also be administered parenterally, that is, subcutaneously, intravenously, intramuscularly, or interperitoneally, as injectable dosages of the compound in a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,3-dioxolane-4-methanol, ethers such as poly(ethyleneglycol) 400, an oil, a fatty acid, a fatty acid ester or glyceride, or an acetylated fatty acid glyceride with or without the addition of a pharmaceutically acceptable surfactant such as a soap or a detergent, suspending agent such as pectin, carbomers, methylcellulose, hydroxypropylmethylcellulose, or carboxymethylcellulose, or emulsifying agent and other pharmaceutically adjuvants.

Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum, and mineral oil. Suitable fatty acids include oleic acid, stearic acid, and isostearic acid. Suitable fatty acid esters are, for example, ethyl oleate and isopropyl myristate. Suitable soaps include fatty alkali metal, ammonium, and triethanolamine salts and suitable detergents include cationic detergents, for example, dimethyl dialkyl ammonium halides, alkyl pyridinium halides, and alkylamines acetates; anionic detergents, for example, alkyl, aryl, and olefin sulfonates, alkyl, olefin, ether, and monoglyceride sulfates, and sulfosuccinates; nonionic detergents, for example, fatty amine oxides, fatty acid alkanolamides, and polyoxyethylenepolypropylene copolymers; and amphoteric detergents, for example, alkyl-beta-aminopropionates, and 2-alkylimidazoline quaternary ammonium salts, as well as mixtures.

The parenteral compositions of this invention will typically contain from about 0.5% to about 25% by weight of the active ingredient in solution. Preservatives and buffers may also be used advantageously. In order to minimize or eliminate irritation at the site of injection, such compositions may contain a non-ionic surfactant having a hydrophile-lipophile balance (HLB) of from about 12 to about 17. The quantity of surfactant in such formulations ranges from about 5% to about 15% by weight. The surfactant can be a single component having the above HLB or can be a mixture of two or more components having the desired HLB.

Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The pharmaceutical compositions may be in the form of sterile injectable aqueous suspensions. Such suspensions may be formulated according to known methods using suitable dispersing or wetting agents

and suspending agents such as, for example, sodium carboxymethyl-cellulose, methylcellulose, hydroxypropylmethyl-cellulose, sodium alginate, polyvinylpyrrolidone, gum tragacanth and gum acacia; dispersing or wetting agents which may be a naturally-occurring phosphatide such as lecithin, a condensation product of an alkylene oxide with a fatty acid, for example, polyoxyethylene stearate, a condensation product of ethylene oxide with a long chain aliphatic alcohol, for example, heptadecaethyleneoxycetanol, a condensation product of ethylene oxide with a partial ester derived from a fatty acid and a hexitol such as polyoxyethylene sorbitol monooleate, or a condensation product of an ethylene oxide with a partial ester derived from a fatty acid and a hexitol anhydride, for example, polyoxyethylene sorbitan monooleate.

The suspensions may also contain one or more preservatives, for example, ethyl or n-propyl p-hydroxybenzoate; one or more coloring agents; one or more flavoring agents; and one or more sweetening agents such as sucrose or saccharin.

Oily suspensions may be formulated by suspending the active ingredient in a vegetable oil such as, for example, arachis oil, olive oil, sesame oil or coconut oil, or in a mineral oil such as liquid paraffin. The oily suspensions may contain a thickening agent such as, for example, beeswax, hard paraffin or cetyl alcohol. The sterile injectable preparation may also be a sterile injectable solution or suspension in a non-toxic parenterally-acceptable diluent or solvent. Diluents and solvents that may be employed are, for example, water, Ringer's solution, and isotonic sodium chloride solution. In addition, sterile fixed oils are conventionally employed as solvents or suspending media. For this purpose, any bland, fixed oil may be employed including synthetic mono- or diglycerides. In addition, fatty acids such as oleic acid can be used in the preparation of injectables.

A composition of the invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are, for example, cocoa butter and polyethylene glycol.

Dispersible powders and granules are suitable for the preparation of an aqueous suspension. They provide the active ingredient in admixture with a dispersing or wetting agent, a suspending agent and one or more preservatives. Suitable dispersing or wetting agents and suspending agents are exemplified by those already mentioned above. Additional excipients, for example those sweetening, flavoring, and coloring agents described above, may also be present.

The compounds of this invention may be formulated as solutions, suspensions, emulsions, powders, and semisolid preparations administered as an aerosol preparation by means of a pressurized aerosol container together with a gaseous or liquefied propellant such as, for example, dichlorodifluoromethane, dichlorodifluoromethane with dichlorodifluoroethane, carbon dioxide, nitrogen, propane, or the like, with the usual adjuvants such as co-solvents and wetting agents, as may be necessary or desirable. The compounds may also be administered in a non-pressurized form such as in a nebulizer or atomizer. The aerosols are intended for administration as fine, solid particles or as liquid mists via the respiratory system, and the particle size of aerosol preparations intended for administration to the lungs should be below 50 micrometers, in most instances.

The pharmaceutical compositions of the invention may also be in the form of oil-in-water emulsions. The oily phase may be a vegetable oil such as liquid paraffin or a mixture of vegetable oils. Suitable emulsifying agents may be (1) naturally-occurring gums such as gum acacia and gum tragacanth, (2) naturally-occurring phosphatides such as soy bean and lecithin, (3) esters or partial esters derived from fatty acids and hexitol anhydrides, for example, sorbitan monooleate, (4) condensation products of said partial esters with ethylene oxide, for example, polyoxyethylene sorbitan monooleate. The emulsions may also contain sweetening and flavoring agents.

Syrups and elixirs may be formulated with sweetening agents such as, for example, glycerol, propylene glycol, sorbitol or sucrose. Such formulations may also contain a demulcent, and preservative and flavoring and coloring agents.

The compositions of the invention can also contain other conventional pharmaceutically-acceptable compounding ingredients, generally referred to as carriers or diluents, as necessary or desired. Any of the compositions of this invention may be preserved by the addition of an antioxidant such as ascorbic acid or by other suitable preservatives. Conventional procedures for preparing such compositions in appropriate dosage forms can be utilized.

The following specific examples are presented to illustrate compositions of this invention, but they should not be construed as limiting the scope of this invention in any way.

EXAMPLE 17

| | |
|---|---|
| A tablet is prepared from 2-fluoromethyltyrosine | 250 mg |
| Starch | 40 mg |
| Talc | 10 mg |
| Magnesium | 10 mg |

EXAMPLE 18

| | |
|---|---|
| A capsule is prepared from 2-fluoromethyltyrosine | 400 mg |
| Talc | 40 mg |
| Sodium Carboxymethyl Cellulose | 40 mg |
| Starch | 120 mg |

It should be apparent to one of ordinary skill in the art that changes and modifications can be made to this invention without departing from the spirit or scope of the invention as it is set forth herein.

**Claims**

**1.** A compound of the formula

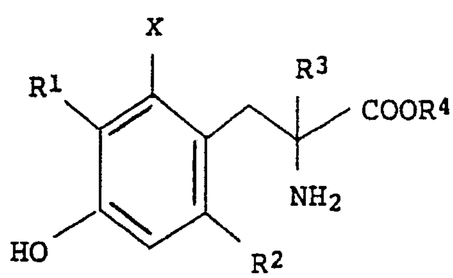

(I)

wherein
X is $CH_2F$ or $CHF_2$,
$R^1$ and $R^2$ are each independently hydrogen, fluorine, chlorine, bromine or iodine, and when one of either $R^1$ or $R^2$ is a halogen then the other must be hydrogen,
$R^3$ is hydrogen or methyl,
$R^4$ is hydrogen, $(C_1\text{-}C_4)$alkyl, phenyl or benzyl when X is $CHF_2$, and $R^4$ is hydrogen when X is $CH_2F$,
and the salts thereof.

**2.** A compound according to claim 1 wherein X is $CH_2F$.

**3.** A compound according to claim 1 wherein X is $CHF_2$.

**4.** A compound according to either claim 2 or claim 3 wherein $R_1$ is fluoro.

**5.** A compound according to either claim 2 or claim 3 wherein $R_2$ is fluoro.

**6.** A compound according to either claim 2 or claim 3 wherein $R^1$ and $R^2$ are each hydrogen.

**7.** A compound according to either claim 2 or claim 3 wherein $R^1$ is chloro.

**8.** A compound according to either claim 2 or 3 wherein $R^2$ is chloro.

**9.** A compound according to claim 3 wherein $R^4$ is hydrogen or $(C_1\text{-}C_4)$alkyl.

**10.** A compound according to claim 2 which is 2-fluoromethyltyrosine or alpha-methyl-2-fluoromethyltyrosine.

**11.** A compound according to claim 3 which is 2-difluoromethyltyrosine or alpha-methyl-2-difluoromethyltyrosine.

**12.** A compound according to claim 4 which is 2-fluoromethyl-3-fluoro-p-tyrosineor 2-difluoromethyl-3-fluoro-p-tyrosine.

**13.** A compound according to claim 5 which is 2-fluoromethyl-6-fluoro-p-tyrosineor 2-difluoromethyl-6-fluoro-p-tyrosine.

**14.** A pharmaceutical composition comprising a pharmacologically effective amount of a compound of formula I, or a salt thereof, and a pharmaceutically acceptable carrier.

**15.** Use of the compound of claims 1 to 14 as pharmaceutically active compound.

**16.** Use of the compound of claims 1 to 14 for the preparation of a medicine useful for the treatment of diseases in which the inhibition of tyrosine hydroxylase is needed.

European Patent Office

# EUROPEAN SEARCH REPORT

Application Number

EP 90 40 1022

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-4 879 398 (D.P. GETMAN) * Column 10, line 51 - column 14, line 39 * | 1 | C 07 C 229/36 A 61 K 31/195 |
| A | EP-A-0 027 993 (MERCK & CO. INC.) * Page 2, line 1 - page 4, line 4 * | 1 | |
| A | EP-A-0 040 150 (MERELL TORAUDE ET COMPAGNIE) * Page 3, line 25 - page 5, line 23 * | 1 | |
| A | EP-A-0 007 600 (MERCK & CO. INC.) * Page 2, line 11 - page 4, line 32 * | 1 | |

TECHNICAL FIELDS SEARCHED (Int. Cl.5)

C 07 C 229/00
A 61 K 31/00

The present search report has been drawn up for all claims

| Place of search | Date of completion of search | Examiner |
|---|---|---|
| The Hague | 06 December 90 | PAUWELS G.R.A. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document